# EUROPEAN PATENT SPECIFICATION

(11) **EP 0 552 624 B1**
(45) Date of publication and mention of the grant of the patent: **28.06.2000**
(21) Application number: 93100257.0
(22) Date of filing: 11.01.1993
(51) Int. Cl.: A61K 31/20, A61K 31/07

(54) **Use of 9-cis retinoic acid, salts and esters thereof for the treatment of epithelial tumors**
Verwendung von 9-cis-Retinsäure sowie ihre Salze und Ester, zur Behandlung von Epitheltumore
Utilisation de l'acide 9-cis-rétinoique, ses sels et esters, pour le traitement de tumeurs épithéliales

(30) Priority: 22.01.1992 US 823741; 22.01.1992 US 823786; 22.01.1992 US 823928; 23.01.1992 US 824647
(43) Date of publication of application: 28.07.1993
(73) Proprietor: F. HOFFMANN-LA ROCHE AG, 4002 Basel (CH)
(72) Inventor: Bollag, Werner, CH-4054 Basle (CH); Grippo, Joseph Francis, Basking Ridge, N.J. 07920 (US); Levin, Arthur, Glen Ridge, N.J. 07028 (US)
(74) Representative: Kjellsaa-Berger, Hanny, Dr.

(56) References cited:
- WO-A-88/07857
- WO-A-90/06751
- WO-A-93/11755
- DE-A- 3 827 467
- CHEMICAL ABSTRACTS, vol. 118, no. 5, 1 February 1993, Columbus, Ohio, US; abstract no. 39207c, TOYODA HITOSHI ET AL. 'PREPARATION OF VITAMIN A ACID ESTER COMPOUNDS AS ANTITUMOR AGENTS AND ULCER THERAPEUTICS FOR SKIN AND DIGESTIVE TRACT' page 717 ;column 2 ;
- LEUKEMIA RESEARCH vol. 10, no. 6, 1986, pages 619 - 629 A. YEN ET AL 'RETINOIC ACID INDUCED HL-60 MYELOID DIFFERENTIATION: DEPENDENCE OF EARLY AND LATE EVENTS ON ISOMERIC STRUCTURE'
- J. AM. ACAD. DERMATOL. vol. 6, 1982, pages 824 - 827 F.L. MEYSKENS 'STUDIES OF RETINOIDS IN THE PREVENTION AND TREATMENT OF CANCER'
- THE LANCET vol. 2, 1980, pages 224 - 226 LEVINE ET AL. 'TOPICAL VITAMIN-A-ACID THERAPY FOR CUTANEOUS METASTATIC MELANOMA'
- J. AM. ACAD. DERMATOL. vol. 9, no. 5, 1983, pages 797 - 805 W. BOLLAG ET AL. 'THE DEVELOPMENT OF RETINOIDS IN EXPERIMENTAL AND CLINICAL ONCOLOGY AND DERMATOLOGY'
- JOURNAL OF CHROMATOGRAPHY vol. 227, no. 2, 1982, pages 471 - 484 F.M. VANE ET AL. 'DETERMINATION OF 13-CIS-RETINOIC ACID AND ITS MAJOR METABOLITE, 4-OXO-13-CIS-RETINOIC ACID, IN HUMAN BLOOD BY REVERSED-PHASE HIGH-PERFORMANCE LIQUID CHROMATOGRAPHY'
- NATURE vol. 355, no. 6358, 23 January 1992, pages 359 - 361 A.A. LEVIN ET AL. '9-CIS RETINOIC ACID STEREOISOMER BINDS AND ACTIVATES THE NUCLEAR RECEPTOR RXRa'
- DOWLATSHAHI K, MEHTA RG, THOMAS CF, DINGER NM, MOON RC. Therapeutic effect of N-(4-hydroxyphenyl)retinamide on N-methyl-N-nitrosourea-induced rat mammary cancer. Cancer Letters 1989, vol. 47,
- MICHNA H, GEHRING S, KUEHNEL W, NISHINO Y, SCHNEIDER MR. The antitumor potency of progesterone antagonists is due to their differentiation potential. J Steroid Biochem 1992, vol. 43, No. 1-3,
- MCCORMICK DL, ADAMOWSKI CB, FIKS A, MOON RC. Liftetime dose-response relationships for mammary tumor induction by a single adminsitration of N-Methyl-N-nitrosourea. Cancer Research, May 1981,
- INOUE K, FUJIMOTOT S, OGAWA M. Antitumor efficacy of seventeen anticancer drugs in human breast cancer xenografts (MX-1) transplanted in nude mice. Cancer Chemotherapy and Pharmacology, 1983,
- BERGER DP, FIEBIG HH, WINTERHALTER BR, WALLBRECHER E, HENSS H. Preclinical phase II study of ifosfamide in human tumour xenografts in vivo. Cancer Chemotherapy and Pharmacology, 1990, vol. 26,

## Description

The present invention is concerned with the use of 9-cis retinoic acid, its pharmaceutically acceptable salts and its pharmaceutically acceptable hydrolyzable esters in the manufacture of a medicament for medical indications as defined below.

It has been found that 9-cis retinoic acid, its pharmaceutically acceptable salts or its pharmaceutically acceptable hydrolyzable esters possess with regard to epithelial lesions, antihyperplastic, antimetaplastic, antineoplastic tumor-preventative and tumor-therapeutic properties exhibiting limited toxicity or other adverse effects associated with retinoids and that pathological conditions treatment of which involves the above properties can be effectively treated by administering 9-cis retinoic acid, its pharmaceutically acceptable salts or its pharmaceutically acceptable hydrolyzable esters either systemically or topically.

Accordingly, the invention is concerned with the use of 9-cis retinoic acid, its pharmaceutically acceptable salts and its pharmaceutically acceptable hydrolyzable esters for the manufacture of a medicament for the treatment of premalignant or precancerous epithelial lesions or epithelial tumors, for the treatment of leukoplakias of the oral cavity, or for the treatment of epithelial tumors of the breast, cervix, prostate, colon, bladder, esophagus, stomach, larynx, or oral cavity.

The document, J.Am.Acad.Dermatol. vol. 6, 1982, pages 824-827 discloses the use of several synthetic retinoids (all-trans RA, 13-cis RA) for the treatment of cancer. The document, Leukemia Research, vol. 10, no. 6, 1986, pages 619-629 discloses the effects of several synthetic retinoids (including all-trans and 9-cis RA) on the differentiation of HL-60 cells (a test model for acute promyelocytic leukemia).

According to the invention, 9-cis retinoic acid its pharmaceutically acceptable salts or its pharmaceutically acceptable hydrolyzable esters is used for the manufacture of a medical composition which can be administered either systemically or topically. In preparing such composition, any conventional pharmaceutically acceptable carrier can be utilized. When the drug is administered orally, it is generally administered at regular intervals, conveniently at mealtimes or once daily. It has been established that this compound is relatively non-toxic when given topically and when given orally.

Examples of conditions involving premalignant and precancerous epithelial lesions or tumors which are effectively treated with 9-cis retinoic acid are actinic keratoses, arsenic keratoses, xeroderma pigmentosum, Bowen's disease, leukoplakias, metaplasias, dysplasias and papillomas of mucous membranes, e.g. of the mouth, tongue, pharynx and larynx, precancerous changes of the bronchial mucous membrane such as metaplasias and dysplasias (especially frequent in heavy smokers and people who work with asbestos and/or uranium), dysplasias and leukoplakias of the cervix uteri, vulval dystrophy, precancerous changes of the bladder, e.g. metaplasias and dysplasias, papillomas of the bladder as well as polyps of the intestinal tract. Examples of tumors or carcinomas of semi-malignant or malignant nature, of the epithelial origin which are effectively treated by 9-cis retinoic acid are breast tumors, cervical and prostate tumors, bladder tumors, e.g. superficial bladder carcinomas, colon tumors, esophageal tumors, stomach tumors, and laryngeal tumors tumors.

The treatment of precancerous lesions and malignant tumors of epithelial nature can be effected with 9-cis retinoic acid its pharmaceutically acceptable salts or its pharmaceutically acceptable hydrolyzable esters alone or in combination with other measures such as surgery, radiation therapy, hormone therapy or treatment with standard chemotherapy (cytostatic and cytotoxic agents) or biological response modifiers (interferons, interleukins or other cytokines).

In accordance with this invention the 9-cis retinoic acid can be used in the form of its pharmaceutically acceptable hydrolyzable esters. Any pharmaceutically acceptable hydrolyzable ester can be used according to this invention. Among the esters are the aromatic esters such as benzyl (OBzl) or benzyl substituted with lower alkyl, halo, nitro; or lower alkyl, e.g., t-butyl; or cyclopentyl, cyclohexyl, cycloheptyl; or 9-fluorenylmethyl.

In accordance with this invention, 9-cis retinoic acid or its pharmaceutically acceptable salts and its pharmaceutically acceptable hydrolyzable esters can be provided in pharmaceutically acceptable oral, or topical composition. These pharmaceutical compositions of the invention contain said 9-cis retinoic acid or its pharmaceutically acceptable salts and its pharmaceutically acceptable hydrolyzable esters in association with a compatible pharmaceutically acceptable carrier material. Any conventional carrier material can be utilized. The carder material can be an organic or inorganic inert carrier material suitable for oral administration. Suitable carriers include water, gelatin, gum arabic, lactose, starch, magnesium stearate, talc, vegetable oils, polyalkylene-glycols, petroleum jelly and the like. Furthermore, the pharmaceutical preparations may contain other pharmaceutically active agents. Additional additives such as flavoring agents, preservatives, stabilizers, emulsifying agents, buffers and the like may be added in accordance with accepted practices of pharmaceutical compounding.

The pharmaceutical preparations can be made up in any conventional form including: (a) a solid form for oral administration such as tablets, capsules, pills, powders, granules, and the like; and (b) preparations for topical administrations such as solutions, suspensions, ointments, creams, gels, micronized powders, aerosols and the like. The pharmaceutical preparations may be sterilized and/or may contain adjuvants such as preservatives, stabilizers, wetting agents, emulsifiers, salts for varying the osmotic pressure and/or buffers.

For topical administration to the skin the aforementioned compound is preferably prepared as ointments, tinctures, creams, gels, solutions, lotions, sprays, suspensions, shampoos, hair soaps, perfumes and the like. In fact, any conventional composition utilized for application to the scalp or skin can be utilized in accordance with this invention. Among the preferred methods of applying the composition containing the agents of this invention is in the form of a gel, lotion and cream. The pharmaceutical preparation for topical administration to the skin can be prepared by mixing the aforementioned active ingredient with non-toxic, therapeutically inert, solid or liquid carriers customarily used in such preparations. These preparations should contain at least about 0.01 percent by weight, of the active ingredient based upon the total weight of the composition. Since the active ingredient, 9-cis retinoic acid is relatively non-toxic and non-irritating it may be used in topical compositions in amounts exceeding 0.15% percent. It is preferred that these preparations contain about 0.01 to 0.15% percent by weight of the active ingredient based upon the total weight of the composition. It is also preferred to apply these preparations once or twice daily to the skin. These preparations can be applied according to the need of the patient. In carrying out this invention, the active ingredient can be applied in an aqueous solution or an alcohol solution such as ethyl alcohol.

In preparing the topical preparations described above additives such as preservatives, thickeners, perfumes and the like conventional in the art of pharmaceutical compounding of topical preparation can be used. In addition, conventional antioxidants or mixtures of conventional antioxidants can be incorporated into the topical preparations containing the aforementioned active agent. Among the conventional antioxidants which can be utilized in these preparations are included N-methyl-α-tocopherolamine, tocopherols, butylated hydroxyanisole, butylated hydroxytoluene, ethoxyquin and the like. Cream-base pharmaceutical formulations containing the active agent, used in accordance with this invention, are composed of aqueous emulsions containing a fatty acid alcohol, semi-solid petroleum hydrocarbon, 1,2-ethyleneglycol and an emulsifying agent.

Ointment formulations containing the active agent in accordance with this invention comprise admixtures of a semi-solid petroleum hydrocarbon with a solvent dispersion of the active material. Cream compositions containing the active ingredient for use in this invention preferably comprise emulsions formed from a water phase of a humectant, a viscosity stabilizer and water, an oil phase of a fatty acid alcohol, a semi-solid petroleum hydrocarbon and an emulsifying agent and a phase containing the active agent dispersed in an aqueous stabilizer-buffer solution. Stabilizers may be added to the topical preparation. Any conventional stabilizer can be utilized in accordance with this invention. In the oil phase, fatty acid alcohol components function as a stabilizer. These fatty acid alcohol components are derived from the reduction of a long-chain saturated fatty acid of at least about 14 carbon atoms. Also, conventional perfumes and lotions generally utilized in topical preparation for the hair can be utilized in accordance with this invention. Furthermore, if desired, conventional emulsifying agents can be utilized in the topical preparations of this invention.

A preferred oral dosage form comprises tablets, capsules of hard or soft gelatin, methylcellulose or of another suitable material easily dissolved in the digestive tract. The oral dosages contemplated in accordance with the present invention will vary in accordance with the needs of the individual patient as determined by the prescribing physician. Generally, however, a daily dosage of from about 0.01 mg to about 3 mg per kg of body weight and preferably from about 0.025 mg to about 1.5 mg per kg of body weight of the patient is utilized. This dosage may be administered according to any dosage schedule determined by the physician in accordance with the requirements of the patient.

It is likewise within the purview of the present invention to incorporate the therapeutically active substance enumerated herein in any desired amount for enteral administration within the oral unit dosage form. It is preferred, however, to formulate preparations containing the active substance of the present invention in such a manner that each dose forms contains from about 1 mg to about 50 mg with suitable therapeutically inert fillers and diluents. It is especially preferred to incorporate such a dosage into soft gelatin capsules and tablets.

The efficacy of 9-cis retinoic acid in the use in accordance with this invention can be demonstrated in test models as described below.

### A. Antitumor effects of 9-cis retinoic acid on human tumor cells lines and on experimental tumor

### Inhibitory effect on growth and proliferation of human transformed epithelial cell lines

9-cis retinoic acid was tested on the human tumor cell lines SCC 15 (squamous cell carcinoma of the tongue) and A 431 (squamous cell carcinoma of the vulva). Proliferation was measured by the capacity of viable cells to reduce MTT dye [3-(4,5-dimethylthiazol-2-yl)-2,5-diphenyltetrazoliumbromide] by a mitochondrial enzymatic activity (Mosmann T. Rapid colorimetric assay for cellular growth and survival: application to proliferation and cytotoxic assays. J. Immunol. Methods 65: 55-63, 1983).

### Results

### Human squamous cell carcinoma SOC 15 (tongue)

| - Viable cells in % of controls after 7 days of incubation | |
|---|---|
| Controls (DMSO) | 100 ± 11.4 |
| 9-cis retinoic acid (3·10⁻⁷ M) | 54 ± 7.5 p < 0.0025 |
| interferon α (IFNα) (1000 U/ml) | 60 ± 8.3 p < 0.005 |
| 9-cis retinoic acid + IFNα | 20 ± 2.5 p < 0.001 |

9-cis retinoic acid in a concentration of 3·10⁻⁷ M inhibited proliferation by 46%, in combination with IFNα even by 80%.

### Human squamous cell carcinoma A 431 (vulva)

| - Viable cells in % of controls after 7 days of incubation | |
|---|---|
| Controls (DMSO) | 100 ± 8.7 |
| 9-cis retinoic acid (3·10⁻⁷ M) | 36 ± 1.8 p < 0.001 |
| IFNα (1000 U/ml) | 42 ± 0.9 p < 0.0005 |
| 9-cis retinoic acid + IFNα | 23 ± 1.4 p < 0.001 |

9-cis retinoic acid in a concentration of 3·10⁻⁷ M inhibited proliferation by 64%, in combination with IFNα even by 77%.

9-cis retinoic acid had a marked growth-inhibitory effect on the two tested human epithelial cancer cell lines.

9-cis retinoic acid was further investigated on its effect on other transformed cell lines. A proliferation assay was performed on a virus containing cell line. SKv-l1, a transformed keratinocyte cell line containing human papilloma virus 16 (HPV 16) was tested with regard to inhibition of proliferation by 9-cis retinoic acid in two experiments with four replicates. 5·10⁴ cells/well were plated in Eagle's medium and incubated for 2 days. Direct cell counting with a hemocytometer was done before and after incubation with the retinoid in a concentration of 10⁻⁶ M and in corresponding DMSO controls. Growth inhibition was expressed in % compared to controls.

Result: 9-cis retinoic acid in a concentration of 10⁻⁶ M significantly inhibited proliferation of the human transformed keratinocytes SKv-l1 by 32.4% (p < 0.001).

### B. Effect of 9-cis retinoic acid on tumor cell induced angiogenesis

It is now cell established that unrestricted growth of tumors is dependent on angiogenesis. Increase in tumor cell population must be preceded by an increase in new capillaries that converge upon the tumor. Therefore by inhibiting angiogenesis, tumor growth is inhibited (Folkman J. How is blood vessel growth regulated in normal and neoplastic tissue? G.H.A. Clowes Memorial Award Lecture. Cancer Res. 46: 467-473, 1986; Sidky YA and Borden EC. Inhibition of angiogenesis by interferons: Effects on tumor- and lymphocyte-induced vascular responses. Cancer Res. 47: 5155-5161, 1987).

The angiogenesis assays have been, previously described (Sidky YA and Auerbach R. Lymphocyte induced angiogenesis: A quantitative and sensitive assay of the graft-vs-host reaction. J. exp Med 141: 1084-1100, 1975. Majewski S et al. Inhibition of tumour-induced angiogenesis by systemically administered protamine sulphate. Int J Cancer 33: 831-833, 1984). Two assays were used in testing 9-cis retinoic acid. In the first assay the retinoid was added in vitro to the cell line before being injected into mice. In the second assay the mice were injected intraperitoneally with the retinoid in vivo before the tumor cell line was injected intradermally.

Method: Cells of two tumorigenic cell lines, SKv-e2 and HeLa, and of a non-tumorigenic cell line, SKv-e1 were preincubated in vitro for 48 hours with 9-cis retinoic acid at a concentration of 10⁻⁷ M followed by cell counting and washing in TC 199 medium. The cell concentration was adjusted to 2·10⁵ viable cells per 0.1 ml of medium and injected intradermally into X-ray irradiated, but not retinoid-treated Balb/c mice. Controls were injected with tumor cells preincubated with DMSO. The angiogenic effect was determined according to the methods referred to above.

### Results

a) Effect of in vitro preincubation with 9-cis retinoic acid on angiogenic capacity of the cell lines SKv-e2 (tumorigenic), HeLa (tumorigenic) and SKv-e1 (non-tumorigenic), in Balb/c mice

| | Mean number of blood vessels | | |
|---|---|---|---|
| | SKv-e2 | HeLa | SKv-e1 |
| Controls | 34.7±3.7 | 32.5±2.7 | 26.3±1.5 |
| 9-cis retinoic acid | 23.6±4.2 p < 0.001 | 23.8±2.9 p < 0.001 | 25.6±2.2 p < 0.1 |

In contrast to the inhibitory effect of 9-cis retinoic acid on the formation of new blood vessels induced by the two tumorigenic cell lines, 9-cis retinoic acid did not inhibit angiogenesis induced by the non-tumorigenic subline.
b) Inhibition of cell-induced angiogenic effect by pretreatment of Balb/c mice in vivo by 9-cis retinoic acid.

| | Mean number of blood vessels | | |
|---|---|---|---|
| | Control | 9-cis retinoic acid | |
| HeLa (Tumorigenic) | 28.3 ± 0.8 | 16.3 ± 1.8 | p < 0.001 |
| SKv-12 (Tumorigenic) | 30.3 ± 2.6 | 15.3 ± 1.0 | p < 0.001 |
| SKv-e2 (Tumorigenic) | 31.3 ± 2.7 | 19.7 ± 0.6 | p < 0.001 |
| SKv-e1 (Non-Tumorigenic) | 19.7 ± 3.7 | 24.3 ± 5.6 | p > 0.1 |

Mice treated with 9-cis retinoic acid showed an inhibition of new blood vessel formation when injected with cells of 3 tumorigenic cell lines. There was no anti-angiogenic effect when a non-tumorigenic cell line was used.

9-cis retinoic acid inhibits in vitro and in vivo the formation of new blood vessels induced by tumor cell lines. Since neovascularisation is a prerequisite for tumor growth, 9-cis retinoic acid acts not only by a direct antitumor effect via inhibition of proliferation and/or induction of differentiation but also by an anti-angiogenic effect, inhibiting indirectly tumor growth.

### C. Effect of 9-cis retinoic acid on chemically-induced skin papillomas in mice

The preventive and therapeutic effect of retinoids on chemically-induced tumors of the skin, of the respiratory, digestive and urinary tract as well as of the mammary gland is well established (Bollag W. and Hartmann HR. Prevention and therapy of cancer with retinoids in animal and man. Cancer Surveys 2: 293-314, 1983). 9-cis retinoic acid has a marked therapeutic effect on established skin papillomas of mice leading to regression of these epithelial tumors. The papilloma test is a standard test for the antitumor screening of retinoids.

### Results

| Dose mg/kg i.p. | Percent change in the average sum of the papilloma diameters per mouse | |
|---|---|---|
| Controls | + 23.5 ± 2.1 | |

| 9-cis retinoid acid | | |
|---|---|---|
| 400 | - 43.3 ± 2.4 | p < 0.0005 |
| 200 | - 24.8 ± 4.5 | p < 0.0005 |

9-cis retinoic acid had a substantial therapeutic antitumor effect on established chemically-induced skin papillomas of mice, producing regression of this epithelial tumor.

### D. Acute Toxicity

Acute Toxicity was determined in Füllinsdorf albino mice. 9-cis retinoic acid was suspended in rape oil and administered intraperitoneally in one single dose. The lethal dose (LD) of 10%, 50% and 90% of the mice was recorded 24 hours, 10 days and 20 days after the application of the single dose. For each group 10 mice were used.

### Results (in mg/kg of the administered compound)

| | 24 hours | 10 days | 20 days |
|---|---|---|---|
| LD 10% | > 4000 | 1200 | 1200 |
| LD 50% | > 4000 | 1400 | 1400 |
| LD 90% | > 4000 | 1800 | 1800 |

The following examples illustrate pharmaceutical preparations containing the 9-cis retinoic acid as provided by the present invention. The compound 9-cis retinoic acid can also be designated by the name (E,Z,E,E)-3,7-dimethyl-9-[2,6,6-trimethyl-1-cyclohexen-1-yl]-2,4,6,8-nonatetraenoic acid.

### Example 1

| Lotion (solution) | | preferred |
|---|---|---|
| 9-cis Retinoic Acid | 0.02 - 0.30 g | |
| Propylene Glycol | 5.00 - 20.00 g | 10.00 g |
| PEG-Glyceryl Cocoate * | 0.00 - 20.00 g | 10.00 g |
| dl-α-Tocopherol | 0.001 - 0.50 g | 0.02 g |
| Ascorbyl Palmitate | 0.01 - 0.20 g | 0.10 g |
| Propyl Gallate | 0.001 - 0.02 g | 0.002 g |
| Citric acid, anhydr ** | 0.00 - 0.20 g | 0.01 g |
| Isopropanol *** | 40.00 - 90.00 g | 50.00 g |
| Water, ad | 100.00 g | 100.00 g (resp. ml) |

| | | |
|---|---|---|
| * or other tensides | | |
| ** or other complexing agents e.g. EDTA | | |
| *** or other alcohols e.g., Ethanol | | |

### Example 2

| Gel | | preferred |
|---|---|---|
| 9-cis Retinoic Acid | 0.02 - 0.30 g | |
| Propylene Glycol | 5.00 - 20.00 g | 10.00 g |
| PEG-Glyceryl Cocoate * | 0.00 - 20.00 g | 10.00 g |
| dl-α-Tocopherol | 0.001 - 0.50 g | 0.02 g |
| Ascorbyl Palmitate | 0.01 - 0.20 g | 0.10 g |
| Propyl Gallate | 0.001 - 0.02 g | 0.002 g |
| Citric acid, anhydr ** | 0.00 - 0.20 g | 0.01 g |
| Isopropanol *** | 40.00 - 90.00 g | 50.00 g |
| HPMC **** | 0.50 - 5.00 g | 3.00 g |
| Preservative ***** | q.s. | q.s. |
| Water, ad | 100.00 g | 100.00 g |

| | | |
|---|---|---|
| * or other tensides | | |
| ** or other complexing agents e.g. EDTA | | |
| *** or other alcohols e.g., Ethanol | | |
| **** Hydroxypropyl Methylcellulose or other polymers e.g. neutralized Carbomer, Methyl Cellulose, Sodium Carboxymethylcellulose | | |
| ***** Preservatives e.g., Paraben esters (methyl, ethyl, propyl, butyl), Sorbic Acid, Benzoic Acid | | |

### Example 3

| Cream | | preferred |
|---|---|---|
| 9-cis Retinoic Acid | 0.02 - 0.30 g | |
| Glycerol | 0.00 - 10.00 g | 5.00 g |
| Na₂EDTA | 0.001 - 0.50 g | 0.03 g |
| Glycerides * | 5.00 - 20.00 g | 10.00 g |
| Cetyl Alcohol | 0.50 - 5.00 g | 1.00 g |
| Stearyl Alcohol | 0.50 - 5.00 g | 1.00 g |
| Glycerol mono Stearate | 1.00 - 8.00 g | 4.00 g |
| Cetaereth ** | 0.50 - 5.00 g | 2.00 g |
| dl-α-Tocopherol | 0.001 - 0.50 g | 0.02 g |
| Preservative *** | q.s. | q.s. |
| Water, ad | 100.00 g | 100.00 g |

| | | |
|---|---|---|
| * e.g. Caprylic/Capric/Triglyceride, Caprylic/Capric/Linoleic Triglyceride, natural glycericles, as well as e.g., Propylene Glycol, Dicaprylate/Dicaprate and waxes such as Stearyl Stearate, Oleyl Oleate, Isopropyl Myristate. | | |
| ** Ceteareth 5-30, or other emulsifiers such as Polysorbate 20-80, Sorbitane esters of fatty acids, fatty acid esters of PEG. | | |
| *** Preservatives e.g., Paraben esters (methyl, ethyl, propyl, butyl), Sorbic Acid, Benzoic Acid. | | |

### Example 4

### Fill mass for soft gelatin capsules

| | |
|---|---|
| 9-cis Retinoic Acid | 5.00 - 50.00 mg |
| Oil * | 1 - 3 parts |
| Wax mixture ** | 1 - 5 parts |
| Fill volume | 1 - 6 minims (61 - 370 µl) |

| | |
|---|---|
| * natural vegetable oils, e.g., soy oil, peanut oil, and artificial glycerides | |
| ** composition of natural and artificial waxes or partially hydrated fats | |

### Example 5

### 1. Hard Gelatine capsules containing 20 mg active substance:

| Composition: One Capsule contains: | |
|---|---|
| 9-cis Retinoic acid | 20.0 mg |
| Gelatine Bloom 30 | 70.0 mg |
| Maltodextrin MD 05 | 108.0 mg |
| dl-α-Tocopherol | 2.0 mg |
| Sodium ascorbate | 10.0 mg |
| Microcrystalline cellulose | 48.0 mg |
| Magnesium stearate | 2.0 mg |
| (weight capsule content) | 260.0 mg |

### Procedure:

The active substance is wet milled in a solution of gelatine, maltodextrin, dl-α-Tocopherol and sodium ascorbate.

The wet milled suspension is spray-dried.

The spray-dried powder is mixed with microcrystalline cellulose and magnesium stearate.

260 mg each of this mixture are filled into hard gelatine capsules of suitable size and color.

### Example 6

### 2. Tablet containing 20 mg active substance:

| Composition: | |
|---|---|
| Tablet kernel: | |
| 9-cis Retinoic acid | 20.0 mg |
| Anhydrous lactose | 130.5 mg |
| Microcrystalline cellulose | 80.0 mg |
| dl-α-Tocopherol | 2.0 mg |
| Sodium ascorbate | 10.0 mg |
| Polyvinylpyrrolidone K30 | 5.0 mg |
| Magnesium stearate | 2.5 mg |
| (Kernel weight) | 250.0 mg |

| Film coat: | |
|---|---|
| 9Hydroxypropyl methylcellulose | 3.5 mg |
| Polyethylenglycol 6000 | 0.8 mg |
| Talc | 1.3 mg |
| Iron oxide, yellow | 0.8 mg |
| Titanium dioxide | 0.8 mg |
| (weight of the film) | 7.4 mg |

### Procedure:

9-cis Retinoic acid is mixed with anhydrous lactose and microcrystalline cellulose.

The mixture is granulated in water with a solution/dispersion of polyvinylpyrrolidone, dl-α-Tocopherol and sodium ascorbate.

The granular material is mixed with magnesium stearate and afterwards pressed as kernels with 250 mg weight.

The kernels are film coated with a solution/suspension of above-mentioned composition.

### Example 7

### Sachet containing 50 mg active substance:

| Composition: | |
|---|---|
| 9-cis Retinoic acid | 50.0 mg |
| Lactose, fine powder | 990.0 mg |
| Microcrystalline cellulose | 1400.0 mg |
| Sodium Carboxymethyl-cellulose | 14.0 mg |
| dl-α-Tocopherol | 5.0 mg |
| Sodium ascorbate | 20.0 mg |
| Polyvinylpyrrolidone K30 | 10.0 mg |
| Magnesium stearate | 10.0 mg |
| Flavouring Agents | 1.0 mg |
| (Fill weight of a sachet) | 2500.0 mg |

### Procedure:

9-cis Retinoic acid is mixed with lactose, microcrystalline cellulose and sodium carboxymethyl cellulose.

The mixture is granulated in water with a solution/dispersion of polyvinylpyrrolidone, dl-α-Tocopherol and sodium ascorbate.

The granule is mixed with magnesium stearate and flavoring agents.

It is filled into sachets of suitable size.

## Claims

1. The use of 9-cis retinoic acid, its pharmaceutically acceptable salts and its pharmaceutically acceptable hydrolyzable esters for the manufacture of a medicament for the treatment of premalignant or precancerous epithelial lesions or epithelial tumors, for the treatment of leukoplakias of the oral cavity, or for the treatment of epithelial tumors of the breast, cervix, prostate, colon, bladder, esophagus, stomach, larynx, or oral cavity.

2. The use of 9-cis retinoic acid, its pharmaceutically acceptable salts and its pharmaceutically acceptable hydrolyzable esters for the manufacture of a medicament for the treatment of the medical indications as defined in claim 1 wherein said medicament is for oral application.

3. The use of 9-cis retinoic acid, its pharmaceutically acceptable salts and its pharmaceutically acceptable hydrolyzable esters for the manufacture of a medicament for the treatment of the medical indications as defined in claim 1 wherein said medicament is for topical application.

4. The use as in claim 2 wherein a dosage unit of said medicament contains from 1 to 50 mg of active ingredient.

5. The use as in claim 3 wherein said medicament contains from 0.01% to 0.15% by weight of active ingredient.

6. The use as in claim 3 wherein said medicament contains from 0.02% to 0.05% by weight of active ingredient.

## Patentansprüche

1. Verwendung von 9-cis-Retinsäure, ihren pharmazeutisch verträglichen Salzen und ihren pharmazeutisch verträglichen hydrolysierbaren Estern zur Herstellung eines Arzneimittels zur Behandlung von prämalignen oder präkanzerösen epithelialen Läsionen oder epithelialen Tumoren, zur Behandung von Leukoplakien der Mundhöhle, oder zur Behandung von epithelialen Tumoren der Brust, der Cervix, der Prostata, des Colons, der Blase, der Speiseröhre, des Magens, des Kehlkopfs oder der Mundhöhle.

2. Verwendung von 9-cis-Retinsäure, ihren pharmazeutisch verträglichen Salzen und ihren pharmazeutisch verträglichen hydrolysierbaren Estern zur Herstellung eines Arzneimittels zur Behandlung der medizinischen Indikationen wie in Anspruch 1 definiert, wobei das Arzneimittel zur oralen Applikation ist.

3. Verwendung von 9-cis-Retinsäure, ihren pharmazeutisch verträglichen Salzen und ihren pharmazeutisch verträglichen hydrolysierbaren Estern zur Herstellung eines Arzneimittels zur Behandlung der medizinischen Indikationen wie in Anspruch 1 definiert, wobei das Arzneimittel zur topischen Applikation ist.

4. Verwendung nach Anspruch 2, wobei eine Dosiseinheit des Arzneimittels 1 bis 50 mg an Wirkstoff enthält.

5. Verwendung nach Anspruch 3, wobei das Arzneimittel 0,01 Gew.-% bis 0,15 Gew.-% an Wirkstoff enthält.

6. Verwendung nach Anspruch 3, wobei das Arzneimittel 0,02 Gew.-% bis 0,05 Gew.% an Wirkstoff enthält.

## Revendications

1. Utilisation de l'acide 9-cis rétinoïque de ses sels pharmaceutiquement acceptables et de ses esters hydrolysables pharmaceutiquement acceptables pour la préparation d'un médicament visant à traiter les lésions épithéliales ou tumeurs épithéliales prémalignes ou précancéreuses, à traiter les leucoplasies de la cavité orale, ou à traiter les tumeurs épithéliales du sein, de l'utérus, de la prostate, du colon, de la vessie, de l'oesophage de l'estomac, du larynx ou de la cavité orale.

2. Utilisation de l'acide 9-cis rétinoïque, de ses sels pharmaceutiquement acceptables, et de ses esters hydrolysables pharmaceutiquement acceptables pour la préparation d'un médicament visant à traiter les indications médicales comme il est dit dans la revendication 1, ledit médicament étant destiné à l'application orale.

3. Utilisation de l'acide 9-cis rétinoïque, de ses sels pharmaceutiquement acceptables et de ses esters hydrolysables pharmaceutiquement acceptables pour la préparation d'un médicament visant à traiter les indications médicales telles que définies dans la revendication 1, ledit médicament étant conçu pour l'application locale.

4. Utilisation comme dans la revendication 2 dans laquelle une unité posologique dudit médicament contient de 1 à 50 mg d'ingrédient actif.

5. Utilisation selon la revendication 3 dans laquelle ledit médicament contient de 0,01% à 0,15% en poids d'ingrédient actif.

6. Utilisation selon la revendication 3 dans laquelle ledit médicament contient de 0,02% à 0,05% en poids d'ingrédient actif.
